(19) Europäisches Patentamt
European Patent Office
Office européen des brevets

(11) Veröffentlichungsnummer: **0 205 969 B1**

(12) **EUROPÄISCHE PATENTSCHRIFT**

(45) Veröffentlichungstag der Patentschrift: **02.01.92**

(51) Int. Cl.$^5$: **C07D 307/935**, A61K 31/34

(21) Anmeldenummer: **86107223.9**

(22) Anmeldetag: **28.05.86**

Die Akte enthält technische Angaben, die nach dem Eingang der Anmeldung eingereicht wurden und die nicht in dieser Patentschrift enthalten sind.

(54) **7-Oxo-PGI2-Ephedrinsalze, deren Herstellung und diese Verbindungen enthaltende pharmazeutsche Präparate.**

(30) Priorität: **29.05.85 HU 205085**

(43) Veröffentlichungstag der Anmeldung:
**30.12.86 Patentblatt 86/52**

(45) Bekanntmachung des Hinweises auf die Patenterteilung:
**02.01.92 Patentblatt 92/01**

(84) Benannte Vertragsstaaten:
**AT BE CH DE FR GB IT LI LU NL SE**

(56) Entgegenhaltungen:
**EP-A- 0 031 426**
**WO-A-82/01002**
**DE-A- 2 641 091**
**DE-A- 2 850 885**
**US-A- 4 258 199**

(73) Patentinhaber: **CHINOIN Gyogyszer és Vegyészeti Termékek Gyára RT.**
**To utca 1-5**
**H-1045 Budapest IV(HU)**

(72) Erfinder: **Kovács, Gábor, Dr.Dipl.Ing.chem.**
**R na park 4**
**H-1142 Budapest(HU)**
Erfinder: **Galambos, Géza, Dr.Dipl.Ing.chem.**
**Petöfi u. 6**
**H-1195 Budapest(HU)**
Erfinder: **Tömösközi, Istvàn, Dr.**
**Pozsonyi u.6**
**H-1045 Budapest(HU)**
Erfinder: **Kánai, Károly, Dipl.Ing.Chem.**
**Mark u.7**
**H-1055 Budapest(HU)**
Erfinder: **Györy, Péter, Dr.Dipl.Ing.Chem.**
**Gyakorl u.32**
**H-1106 Budapest(HU)**
Erfinder: **Körmöczy, Péter, Dr.**
**Uri u. 33**
**H-1014 Budapest(HU)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**CHEMICAL ABSTRACTS Band 104, Nr. 17, 28. April 1986; Seite 669, Spalte 2, Zusammenfassung Nr. 148622r, Columbus, Ohio, US; & HU - A - 35658 (CHINOIN GYOGYSZER ES VEGYESZETI TERMEKEK GYARA RT.), 29.07.1985; im Zusammenhang mit CHEMICAL SUBSTANCE INDEX, Noc-P, Januar-Juni 1986, Seite 5512CS, Spalte 1, Zeilen 71 - 73, 79 - 83; & EP - A - 01 63905 (Cat. A, D. P)**

Erfinder: **Stadler, István, Dr.**
**Népstadion u.18**
**H-1143 Budapest(HU)**
Erfinder: **Szekeres, Lászlö, Dr.**
**Kazinczy u.2**
**H-6720 Szeged(HU)**
Erfinder: **Papp, Gyula, Dr.**
**Bécsi krt.37-39**
**H-6722 Szeged(HU)**
Erfinder: **Udvary, Eva, Dr.**
**Olajos u. 2/a**
**H-6723 Szeged(HU)**
Erfinder: **Hadházy, Pál, Dr.**
**Baranyai u.31/b**
**H-1117 Budapest(HU)**
Erfinder: **Marton, Jenö, Dr.**
**Szél u.19**
**H-1035 Budapest(HU)**
Erfinder: **Dormán, György, Dipl.Ing.Chem.**
**Kondorasi u. 15**
**H-1116 Budapest(HU)**

(74) Vertreter: **Bartsch, Elisabeth, Dr.**
**Patentanwälte Lotterhos & Partner Lichtensteinstrasse 3**
**W-6000 Frankfurt am Main 1(DE)**

## Beschreibung

Die Erfindung betrifft neue 7-Oxo-$PGI_2$-Ephedrinsalz-analoge, Verfahren zur Herstellung dieser Verbindungen und diese Verbindungen enthaltende pharmazeutische Präparate.

Die neuen Verbindungen der Erfindung zeigen dieselbe Wirksamkeit wie die aus der Literatur bekannten Verbindungen 7-Oxo-16,17,18,19,20-pentanor-15-cyclopentyl- $PGI_2$ und 7-Oxo-$PGI_2$ und deren Salze.

Bei der Synthese von Prostacyclin und ganz allgemein von Prostaglandin-analogen sowie bei der Überführung dieser Verbindungen in pharmazeutische Präparate ist es häufig mit Schwierigkeiten verbunden, diese Verbindungen in gut zu handhabender, möglichst kristalliner Form zu erhalten.

Es ist bekannt, daß diese Verbindungen in einigen Fällen vorteilhaft in Form ihrer Metall- und Ammoniumsalze verwendet werden können (US-PS 3 706 789). Besonders günstig sind die Tris-(hydroxymethyl)-aminomethan- (THAM) -Salze von $PGF_2$ und $PGE_2$ (BE-PS 767 926), und das Natriumsalz des Prostacyclins läßt sich leicht handhaben und gut kristallisieren (EP-A 0 048 107).

In der DE-A 28 50 885 ist vorgeschlagen worden, für die Salzbildung von $PGI_2$-Analogen unter anderem auch Tris-(hydroxymethyl)-amino-methan (THAM) oder Ephedrin zu verwenden, die Herstellung dieser Salze aber nicht beschrieben.

Die in der Literatur beschriebenen Kristallisations-methoden (vgl. auch die oben angegebenen Literaturstellen) sind auf 7-Oxo-$PGI_2$ und deren Derivate nicht anwendbar, weil die gebildeten Salze entweder nicht kristallin oder instabil und schwer zu handhaben sind und weil ihre Reinigung kompliziert ist.

In der WO-A 82/01 002 ist zwar die Herstellung eines 7-Oxo-$PGI_2$-THAM-Salzes beschrieben, Kenndaten über das THAM-Salz fehlen. Es ist noch nicht einmal angegeben, ob das Salz in festem, halbfestem oder flüssigem Zustand bei Raumtemperatur vorliegt.

Untersuchungen haben ergeben, daß die Papaverin-, Triäthylamin-, Dicyclohexylamin-, Tris-(hydroxymethythyl)-aminomethan)- (THAM) -Salze des 7-Oxo-$PGI_2$ mit den üblichen Methoden, unter Anwendung von Wasser, Alkanen, Cycloalkanen, aromatischen Kohlenwasserstoffen, Äthern, Ketonen, Estern oder Alkoholen nicht in kristalliner Form hergestellt werden können,und die gleichen Ergebnisse wurden mit dem Natriumsalz des 7-Oxo-$PGI_2$ erhalten.

Die Calcium- und Magnesiumsalze des 7-Oxo-$PGI_2$, die nach an sich bekannten Methoden (J.Org.Chem. 1983, Seite 5341) isoliert und in Form gelblich-weißer Salze erhalten wurden, unterliegen während der Trocknung bzw. während der Umkristallisation einer erheblichen Zersetzung, und ihre Anwendung in der Praxis ist deshalb mit großen Schwierigkeiten verbunden.

Es wurde überraschender Weise gefunden, daß die 7-Oxo-$PGI_2$-Ephedrinsalz-analogen der allgemeinen Formel I

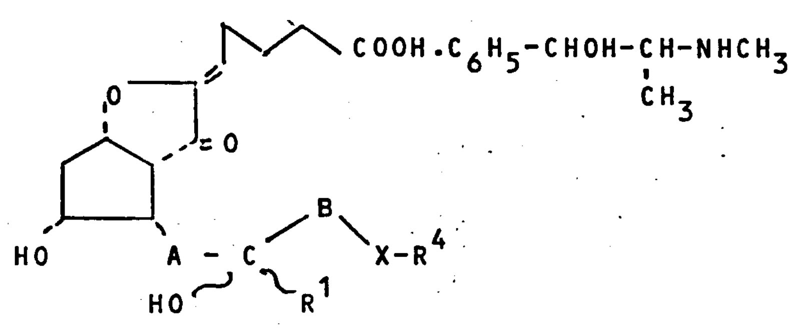

(I) ,

worin

| | |
|---|---|
| A | $-(CH_2)_2$ -, cis- oder trans-CH = CH- oder -C≡C-, |
| $R^1$ | $C_{1-4}$-Alkyl oder Wasserstoff, |
| B | eine Valenzbindung, $-CH_2$ oder $-CR^2R^3$, |
| $R^2$ und $R^3$ | Wasserstoff oder $C_{1-4}$-Alkyl, |
| X | eine Valenzbindung, Sauerstoff oder $-CH_2$ und |
| $R^4$ | $C_{1-6}$-Alkyl. $C_{4-7}$-Cycloalkyl, $C_{1-6}$ $\omega$-Fluoralkyl, $C_{2-6}$-Alkenyl, $C_{2-6}$-Alkinyl, Phenyl oder substituiertes Phenyl |

bedeuten,

äußerst leicht zu handhabende, stabile, in Wasser gut lösliche kristalline Substanzen sind, die sich infolgedessen ausgezeichnet reinigen lassen und als Wirkstoff in pharmazeutischen Präparaten verwendet werden können.

Die leicht zu handhabenden kristallinen Verbindungen der allgemeinen Formel I können weiterhin in die

freien 7-Oxo-prostacyclinsäuren übergeführt und - auf Grund dieser Eigenschaften - bei der Reinigung der Säuren als Zwischenprodukt benutzt werden.

Die 7-Oxo-prostacyclin-Derivate der Erfindung der allgemeinen Formel I stellen stabile Analoge des Prostacyclins ($PGI_2$) dar und sind - im pharmakologischen Profil - dem Natriumsalz des $PGI_2$ ähnlich. Die Pharmakologie und therapeutische Anwendbarkeit des Prostacyclin-Natriumsalzes ist in der wissenschaftlichen Literatur ausführliche beschrieben (Drugs of Today, 19, 605 (1983) und die dort zitierten Literaturstellen).

Die praktische Anwendung des Prostacyclins wird bekanntlich durch die außerordentlich geringe Stabilität dieser Verbindungen erschwert (J.Chem.Soc.Chem.Commun. 1979, 129).

Es sind bereits auf verschiedene Weise stabilisierte Prostacyclin-Derivate bekannt, die eine ähnliche pharmakologische Wirkung aufweisen, und deren Anwendung für therapeutische Zwecke wesentlich einfacher und wirksamer ist (R.F.Newton und Koll. Synthesis 1984, 449; R.Noyori, M.Suzuki, Angew.Chem., Int.Ed.Engl., 23 847 (1984)).

Eine gut bewährte Stabilisierungsmethode betrifft die Stabilisierung der funktionellen Enoläthergruppe - d.h. der empfindlichen Struktureinheit des Prostacyclins - wobei die C-7-Methylengruppe in die Oxogruppe übergeführt wird, was auf Grund der elektronenanziehenden und delokalisierenden Wirkung eine verminderte Empfindlichkeit des Moleküls zur Folge hat (J.Med.Chem. 25, 105 (1982): US-PS 4 330 553: EP-A 0 163 905).

Nach den bekannten Verfahren sind die freien Säuren, die Natriumsalze und die Tris-(hydroxymethyl)-aminomethan-Salze (THAM) der 7-Oxo-prostacyclin-Derivate erhalten worden; diese Verbindungen lassen sich jedoch nur schwer in reiner Form herstellen und bearbeiten.

Die Erfindung betrifft somit neue 7-Oxo-$PGI_2$-Ephedrinsalz-analoge der allgemeinen Formel I

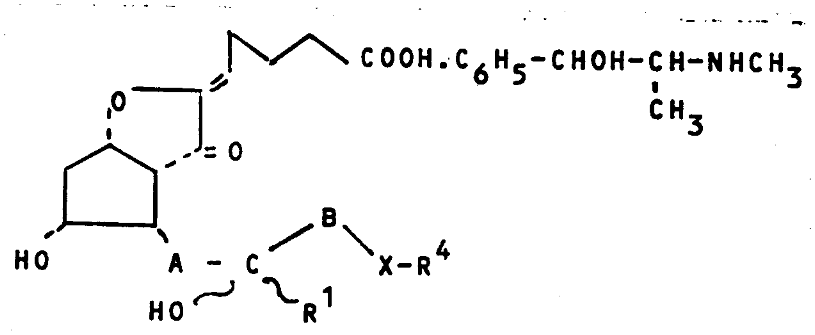

(I) ,

worin
$R^1$, $R^2$, $R^3$, $R^4$, A, B und X die oben angegebene Bedeutung haben.

Die Erfindung betrifft weiterhin ein Verfahren zur Herstellung der neuen 7-Oxo-$PGI_2$-Ephedrinsalz-analogen der allgemeinen Formel I, wonach man

a) aus einer Verbindung der allgemeinen Formel II

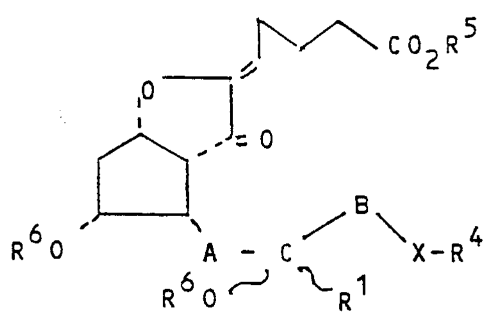

(II) ,

worin
$R^1$, $R^2$, $R^3$, $R^4$, A, B und X die oben angegebene Bedeutung haben,
$R^5$ geradkettiges oder verzweigtes $C_{1-4}$-Alkyl und
$R^6$ geradkettiges oder verzweigtes $C_{1-4}$-Alkanoyl, Benzoyl oder substituiertes Benzoyl bedeuten,
zunächst die Schutzgruppen durch Behandlung mit einer Base und danach die Estergruppe durch

Behandlung mit einer Base in Gegenwart von Wasser entfernt, und dann das so erhaltene Reaktionsgemisch in die Salzbildungsreaktion mit optisch aktivem oder racemischem Ephedrin bei -20 bis +50 °C einführt,
oder
b) aus einer Verbindung der allgemeinen Formel II,
worin
$R^1$, $R^2$, $R^3$, $R^4$, $R^5$, A, B und X die oben angegebene Bedeutung haben und
$R^6$ Wasserstoff ist,
zunächst die Estergruppe durch Behandlung mit einer Base in Gegenwart von Wasser entfernt, und danach das so erhaltene Reaktionsgemisch nach Behandlung mit einer Säure und nach Überführung in einen Extrakt mit einem mit Wasser nicht mischbaren organischen Lösungsmittel in die Salzbildungsreaktion mit optisch aktivem oder racemischem Ephedrin bei -20 bis +50 °C einführt,
oder
c) eine mit einem polaren Lösungsmittel gebildete Lösung einer Verbindung der allgemeinen Formel II,
worin
$R^1$, $R^2$, $R^3$, $R^4$, A, B und X die oben angegebene Bedeutung haben und
$R^5$ und $R^6$ Wasserstoff bedeuten,
bei einer Temperatur im Bereich von -20 bis 50 °C mit optisch aktivem oder racemischem Ephedrin behandelt und danach die so erhaltene Verbindung der allgemeinen Formel I zur Kristallisation bringt.

Die Salzbildung kann in einem polaren Lösungsmittel (z.B. Alkohol, vorzugsweise Äthanol; Ester, z.B. Äthylacetat; Äther, z.B. Diäthyläther; Aceton; Dimethylformamid etc.) vorzugsweise bei Raumtemperatur durchgeführt werden. Das optisch aktive oder racemische Ephedrin wird vorzugsweise in 1,05 bis 5,0, vorzugsweise 1,1, äquivalenter Menge angewendet. Die Kristallisation kann unmittelbar in dem so erhaltenen Gemisch durchgeführt werden; man kann aber auch so verfahren, daß man die Lösung zunächst mit einem Lösungsmittel behandelt, in dem das Ephedrinsalz nur schlecht löslich ist (wie aliphatischen Kohlenwasserstoffen, vorzugsweise Pentan, Hexan, Cyclohexan, aromatischen Kohlenwasserstoffen, z.B. Benzol, Toluol, Xylol, chlorierten Kohlenwasserstoffen, z.B. Chloroform, Tetrachlorkohlenstoff etc.) und danach die Kristallisation durchführt.

Es ist nicht erforderlich, zur Herstellung der Verbindungen der allgemeinen Formel I gereinigte Ausgangsstoffe der allgemeinen Formel II
worin
$R^1$, $R^2$, $R^3$, $R^4$, A, B und X die oben angegebene Bedeutung haben und
$R^5$ und $R^6$ Wasserstoff bedeuten,
einzusetzen.

Werden als Ausgangsstoffe Verbindungen der allgemeinen Formel II,
worin
$R^1$, $R^2$, $R^3$, $R^4$, A, B und X die oben angegebene Bedeutung haben,
$R^5$ $C_{1-4}$-Alkyl ist und
$R^6$ Wasserstoff, geradkettiges oder verzweigtes $C_{1-4}$-Alkanoyl, Benzoyl oder substituiertes Benzoyl bedeuten, verwendet, kann man auch so verfahren, daß man - falls notwendig - die $R^6$ Schutzgruppen nach den in der Literatur angegebenen Methoden mit einer Base (vorzugsweise Alkalicarbonaten, wie Kaliumcarbonat oder Alkalialhoholaten, insbesondere Netriummethylat) unter wasserfreien Bedingungen oder in Gegenwart von Wasser in einem Lösungsmittel (zweckmäßig in einem Alkohol, vorzugsweise in wasserfreiem Methanol) entfernt und danach - falls erforderlich, d.h wenn $R^5$ nicht Wasserstoff ist - die $R^5$-Gruppe nach den in der Literatur angegebenen Methoden, mit Hilfe einer Base (zweckmäßig Alkalihydroxiden, vorzugsweise Natriumhydroxid) in einem mit Wasser mischbaren Lösungsmittel (zweckmäßig Alkoholen vorzugsweise Methanol) in Gegenwart von Wasser abhydrolysiert. Das so erhaltene Reaktionsgemisch wird mit einer anorganischen Säure (zweckmäßig Salzsäure, Schwefelsäure, Phosphorsäure, Natriumbisulfat) auf einen pH-Wert zwischen 3 und 6 angesäuert. Die so erhaltene Lösung der Verbindung der allgemeinen Formel II wird ohne Reinigung in die Salzbildungsreaktion eingeführt; diese Verfahrensstufe wird in der oben beschriebenen Weise durchgeführt.

Unter substituiertem Phenyl oder Benzoyl ist ein Phenyl oder Benzoyl zu verstehen, das durch Halogen substituiertes $C_{1-6}$-Alkyl, Hydroxyl, Sulfhydryl, $C_{1-6}$-Alkanoyloxy-, Benzoyloxy, Amino, Mono- oder Di-$C_{1-6}$-alkylamino, Halogen, $C_{1-6}$-Alkoxy oder $C_{1-6}$-Alkylthio substituiert sein kann.

Die als Ausgangsstoff verwendeten Verbindungen der allgemeinen Formel II sind bekannt. (J.Med.Chem. 25, 105 (1982); US-PS 4 330 553; EP-A 0 163 905).

Die neuen Verbindungen der Erfindung können mit Erfolg zur Reinigung von 7-Oxo-$PGI_2$-analogen benutzt werden; die Verbindungen der Erfindung lassen sich durch Behandlung mit Säuren in die freie

Säure oder durch Einwirkung von Basen in die entsprechenden Salze überführen.

Die pharmakologische Wirksamkeit der erfindungsgemäßen Verbindungen wird nachstehend zusammengefaßt.

Die Verbindungen der Erfindung stimmen im pharmakologischen Profil mit dem von Prostacyclin überein, wobei ihre Wirksamkeit der Aktivität der anderen Formen der entsprechenden 7-Oxo-$PGI_2$-analogen (freie Säure, Natriumsalz) entspricht.

Bei der in vitro bestimmten Blutaggregations-Hemmwirkung des 7-Oxo-16,17,18,19,20-pentanor-15-cyclopentyl-$PGI_2$ gegen ADP (2 $\mu$M) wurde ein $IC_{50}$-Wert von 0,003 nMol/ml sowohl für das bekannte Natriumsalz als auch für das Ephedrinsalz der Erfindung (bestimmt an humanem, an Blutplättchen angereichertem Plasma nach der Methode von Born) erhalten. Ähnliche Werte wurden auch bei der Bestimmung der hämodynamischen Eigenschaften erhalten.

An der Katze bei i.v.-Applikation zeigt das bekannte Natriumsalz eine blutdrucksenkende Wirkung von 21,2 $\mu$g/kg, 40,3 nMol/kg; die entsprechenden Werte des Ephedrinsalzes der Erfindung sind 29,0 $\mu$g/kg, 42,5 nMol/kg ($ED_{50}$).

Diesen Ergebnissen ist zu entnehmen, daß die Verbindungen der allgemeinen Formel I - in der gleichen Dosis angewandt - die gleiche pharmakologische Wirkung wie die anderen Salze aufweisen.

Darüber hinaus hemmen die Verbindungen der allgemeinen Formel I bei Ratten im Magen-Darmtrakt die Magensäuresekretion und üben eine cytoprotektive Wirkung aus, d.h. die Verbindungen der Erfindung reduzieren die Anzahl und mildern die Schwere der Geschwüre, die auf Alkohol, Stress und Indomethacin zurückzuführen sind.

Beim Hund setzen die Verbindungen der allgemeinen Formel I bei Herzinsuffizienz den Sauerstoffverbrauch des Herzens herab; dadurch wird das Entstehen von anginösen Anfällen gehemmt bzw. die Stärke der Anfälle herabgesetzt.

Nach den an Rattenlebern durchgeführten Versuchen eliminieren die Verbindungen der Erfindung die durch Tetrachlorkohlenstoff erzeugten Schädigungen bzw. sie verhindern die Bildung einer Läsion.

Auf Grund des oben angegebenen pharmakologischen Profils können die Verbindungen der allgemeinen Formel I auf allen den therapeutischen Gebieten mit Erfolg eingesetzt werden, auf denen bislang das Prostacyclin-Natriumsalz bzw. die bekannten Salze des 7-Oxo-prostacyclins verwendet worden sind. Bei extrakorporalen Zirkulationen können die Verbindungen der allgemeinen Formel I die Blutaggregation verhindern.

Die Verbindungen der Erfindung können außerdem mit Erfolg zur Verhütung von weiteren Krankheiten - wie pheripheren Gefäßerkrankungen, bei Herzinfarkt zur Reduktion der Stärke des Anfalles - und damit zur Herabsetzung der Sterblichkeitsrate verwendet werden. Die Verbindungen der allgemeinen Formel I eignen sich weiterhin bei bestimmten anginösen Krankheiten zur Herabsetzung der Anzahl und Minderung der Schwere der Anfälle.

Verschiedene weitere cytoprotektive Wirkungen der Verbindungen der Erfindung können zur Behandlung und zur Vorbeugung bei gastrointestinalen Geschwüren (Ulcus) und bei akuten chronischen Leberschädigungen verwendet werden.

Bei Tumoren erhöhen die Verbindungen der allgemeinen Formel I die Überlebensrate infolge Metastase-Hemmwirkung.

Die Erfindung betrifft auch pharmazeutische Präparate, die als Wirkstoff eine oder mehrere Verbindung(en) der allgemeinen Formel I und geeignete, inerte, feste oder flüssige pharmazeutische Träger enthalten.

Die Verbindungen der allgemeinen Formel I können z.B. als Tabletten, Kapseln, flüssige Präparate oder anderen, in der Therapie üblichen Formen fertiggestellt bzw. konfektioniert werden. Die pharmazeutischen Präparate enthalten die üblichen pharmazeutischen Träger, Füllstoffe, Bindemittel usw..

Die pharmazeutischen Präparate der Erfindung können außer der Verbindung der allgemeinen Formel I noch weitere Wirkstoffe enthalten.

Die pharmazeutischen Präparate der Erfindung können nach an sich bekannten Methoden der pharmazeutischen Industrie hergestellt werden und sollen für die intravenöse, subcutane oder orale (gastrointestinale) Applikation verwendet werden. Die anwendbare Dosis hängt von mehreren Faktoren ab (z.B. Alter, Gewicht, Geschlecht der Patienten, Art und Schwere der Krankheit usw.) und liegt im allgemeinen zwischen etwa 0,0001 und etwa 10 mg/kg.

Weitere Einzelheiten der vorliegenden Erfindung sind den nachstehenden Beispielen zu entnehmen, aus denen keine Beschränkungen herzuleiten sind.

Beispiel 1

7-Oxo-prostacyclin-(-)-Ephedrinsalz

(I: A = trans-Vinylen; $R^1$ = H; B = X = Valenzbindung; $R^4$ = n-Pentyl)

1,83 g (5,0 mMol) 7-Oxo-Prostacyclin (II: $R^1$ = $R^5$ = $R^6$ = H; A = trans-Vinylen; B = X = Valenzbindung; $R^4$ = n-Pentyl) löst man in 15 ml wasserfreiem Äthylacetat, gibt dann unter Rühren 0,825 g (5,0 mMol) (-)-Ephedrin oder eine Lösung dieser Base in 5 ml wasserfreiem Äthylacetat zu, rührt das erhaltene Reaktionsgemisch eine Stunde und läßt es danach im Kühlschrank 10 bis 12 Stunden stehen. Das ausgefallene Produkt wird filtriert, mit einer kalten Mischung von Hexan und Äthylacetat (3 : 1) gewaschen und getrocknet. Es werden 1,86 g der im Titel genannten Verbindung erhalten; F.: 121 °C.
Dünnschichtchromatographie nach Ansäuern, in Form der freien Säure:
$R_f$ = 0,34 (Benzol : Dioxan : Essigsäure = 20 : 10 : 1)
$^1$HNMR (Methanol $d_4$): 5,55 (2H, m), 5,31 (1H, t), 5,10 (2H, m), 4,00 (2H, m).

Beispiel 2
7-Oxo-16,17,18,19,20-pentanor-15-cyclopentyl-$PGI_2$-(-)-Ephedrinsalz

(I: A = trans-Vinylen; $R^1$ = H; B = X = Valenzbindung; $R^4$ = Cyclopentyl)

Einer Lösung von 467 mg (1 mMol) 7-Oxo-11,15-diacetyl-16,17,18,19,20-pentanor-15-cyclopentyl-$PGI_2$-methylester (II: $R^5$ = Methyl, $R^6$ = Acetyl; die übrigen Substituenten wie in Titelverbindung) in 25 ml Methanol wird eine methanolische Natriummethylatlösung (0,5 Mol;0,5 mMol;1 M) zugegeben, das erhaltene Reaktionsgemisch eine Stunde bei Raumtemperatur gerührt und teilweise eingeengt.
Dem Rückstand gibt man 3,5 ml einer 1 M Natriumhydroxidlösung zu, rührt das Gemisch eine Stunde bei 40 °C, entfernt das Methanol unter vermindertem Druck, löst den Rückstand in 30 ml Wasser und wäscht ihn zweimal mit je 5 ml Äther. Die ätherische Phase wird verworfen, und die wäßrige Schicht wird mit einer 1 N Natriumbisulfatlösung auf pH 6 angesäuert und zweimal mit je 20 ml Äthylacetat extrahiert. Den pH-Wert der wäßrigen Phase stellt man dann mit 1 N Natriumbisulfatlösung auf 4 ein und extrahiert die Lösung wieder zweimal mit je 20 ml Äthylacetat.
Die vereinigten Äthylacetatphasen werden dann zweimal mit je 10 ml einer gesättigten Natriumchloridlösung extrahiert, etwa 30 Minuten über Magnesiumsulfat gerührt und filtriert. Das Filtrat engt man unter vermindertem Druck auf etwa 5 ml ein, gibt der Lösung 171 mg (0,93 Mol) (-)-Ephedrin zu, läßt das Gemisch 24 Stunden stehen und führt mit Hilfe einer Mischung von Hexan und Äthylacetat die Kristallisation durch. Es werden 113 mg der im Titel genannten Verbindung in Form von weißen Kristallen erhalten, F.: 115 bis 120 °C.
Dünnschichtchromatographie: nach Ansäuern, in Form der freien Säure:
$R_f$ = 0,30 (Benzol : Dioxan : Essigsäure = 20 : 10 : 1)
UV:$\lambda_{max}$ = 288 nm, lg $\epsilon$ = 3,890.

Beispiel 3
7-Oxo-13,14-didehydro-16,17,18,19,20-pentanor-15-cyclohexyl-$PGI_2$-(-)-Ephedrinsalz

(I: A = -C≡C-; $R^1$ = H; B = X = Valenzbindung; $R^4$ = Cyclohexyl)

Einer Lösung von 500 mg (1,1 mMol) 7-Oxo-13,14-didehydro-11,15-diacetyl-16,17,18,19,20-pentanor-15-cyclohexyl-$PGI_2$-methylester (II: $R^5$ = $CH_3$; $R^6$ = Acetyl; die übrigen Substituenten wie in Titelverbindung) in 50 ml Methanol wird eine methanolische Natriummethylatlösung zugegeben (0,5 ml; 0,5 mMol; 1 M), das erhaltene Reaktionsgemisch eine Stunde stehen gelassen und das Methanol unter vermindertem Druck entfernt.
Der Rückstand wird mit 4 ml einer Natriumhydroxidlösung eine Stunde bei 40 °C gerührt und das so erhaltene Reaktionsgemisch, wie in Beispiel 2 angegeben, aufgearbeitet.
Zur Salzbildung werden 180 mg (0,98 mMol) (-)-Ephedrin verwendet. Die Salzbildung wird in der in Beispiel 2 angegebenen Weise durchgeführt, wobei man 218 mg der im Titel genannten Verbindung in Form von weißen Kristallen erhält, F.: 125 bis 128 °C.
Dünnschichtchromatographie: nach Ansäuern, in Form der freien Säure:
$R_f$ = 0,30 (Benzol : Dioxan : Essigsäure = 20 : 10 : 1).

Beispiel 4
7-Oxo-$PGI_2$-Ephedrinsalz

(I: A = trans-Vinylen; $R^1$ = H; B = X = Valenzbindung; $R^4$ = n-Pentyl)

1,1 g (2,9 mMol) 7-Oxo-prostacyclin-methylester (II: $R^5$ = Methyl; $R^6$ = H; die übrigen Substituenten wie in Titelverbindung) löst man in einer Mischung von 10 ml Methanol und 8 ml einer 1 N Natriumhydroxidlösung, rührt das erhaltene Reaktionsgemisch 3 Stunden bei Raumtemperatur und entfernt danach das Methanol unter vermindertem Druck. Den Rückstand löst man in 50 ml Wasser und wäscht ihn zweimal mit je 10 ml Äther. Die wäßrige Phase wird mit 1 N Natriumbisulfatlösung bei 0° C auf pH 4 angesäuert und dreimal mit je 50 ml Äthylacetat extrahiert.
Die vereinigten Äthylacetatphasen werden zweimal mit je 20 ml einer gesättigten Natriumchloridlösung gewaschen, über Natriumsulfat getrocknet und filtriert. Das Filtrat wird auf etwa 5 bis 10 ml eingeengt. Dem Rückstand gibt man 462 mg (2,8 mMol) (-)-Ephedrin zu, läßt das Reaktionsgemisch 24 Stunden stehen und führt mit Hilfe einer Mischung von Hexan und Äthylacetat die Kristallisation durch. Es werden 958 mg der im Titel genannten Verbindung erhalten. Die physikalischen Konstanten des Produktes sind mit denen der nach Beispiel 1 hergestellten Verbindung völlig identisch.

Beispiel 5
7-Oxo-16,16-dimethyl-$PGI_2$-(-)-Ephedrinsalz

(I: A = trans-Vinylen; $R^1$ = H; B = -$CR^2R^3$; X = Valenzbindung; $R^2$ = $R^3$ = $CH_3$; $R^4$ = n-Pentyl)
608 mg (1,5 mMol) 7-Oxo-16,16-dimethyl-$PGI_2$-methylester (II: $R^5$ = $CH_3$; $R^6$ = H; die übrigen Substituenten wie in Titelverbindung) löst man in einer Mischung von 8 ml Methanol und 7 ml einer 1 N Natriumhydroxidlösung und hydrolysiert das erhaltene Reaktionsgemisch eine Stunde bei 40° C. Die weitere Aufarbeitung des Reaktionsgemisches erfolgt in der in Beispiel 4 angegebenen Weise.
Zur Salzbildung werden 231 mg (1,4 mMol) (-)-Ephedrin verwendet. Die Kristallisation wird in Äthylacetat durchgeführt. Es werden 420 mg der im Titel genannten Verbindung in Form von weißen Kristallen erhalten, F.: 113 bis 117° C.
UV:$\lambda_{max}$ = 290 nm, lg $\epsilon$ = 3,885
Dünnschichtchromatographie: nach Ansäuern, in Form der freien Säure:
$R_f$ = 0,48 (Benzol : Dioxan : Essigsäure = 20 : 10 : 1).

**Patentansprüche**

**1.** 7-Oxo-$PGI_2$-Ephedrinsalz-analoge der allgemeinen Formel I

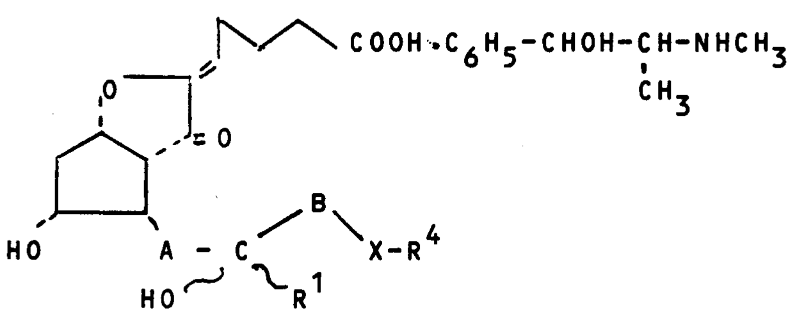

(I) ,

worin

| | |
|---|---|
| A | -$(CH_2)_2$-, cis- oder trans-CH=CH- oder -C≡C-. |
| $R^1$ | $C_{1-4}$-Alkyl oder Wasserstoff, |
| B | eine Valenzbindung, -$CH_2$- oder -$CR^2R^3$, |
| $R^2$ und $R^3$ | Wasserstoff oder $C_{1-4}$-Alkyl, |
| X | eine Valenzbindung, Sauerstoff oder -$CH_2$- und |
| $R^4$ | $C_{1-6}$-Alkyl. $C_{4-7}$-Cycloalkyl, $C_{1-6}$-$\omega$-Fluoralkyl, $C_{2-6}$-Alkenyl, $C_{2-6}$-Alkinyl, Phenyl oder substituiertes Phenyl |

bedeuten.

**2.** 7-Oxo-$PGI_2$-(-)-Ephedrinsalz,
7-Oxo-$PGI_2$-(+)-Ephedrinsalz oder
7-Oxo-$PGI_2$-(±)-Ephedrinsalz.

**3.** 7-Oxo-16,17,18,19,20-pentanor-15-cyclopentyl-$PGI_2$-(-)-Ephedrinsalz,
7-Oxo-16,17,18,19,20-pentanor-15-cyclopentyl-$PGI_2$-(+)-Ephedrinsalz oder

7-Oxo-16,17,18,19,20-pentanor-15-cyclopentyl-$PGI_2$-(±)-Ephedrinsalz.

**4.** Verfahren zur Herstellung von 7-Oxo-$PGI_2$-Ephedrinsalz-analogen der allgemeinen Formel I

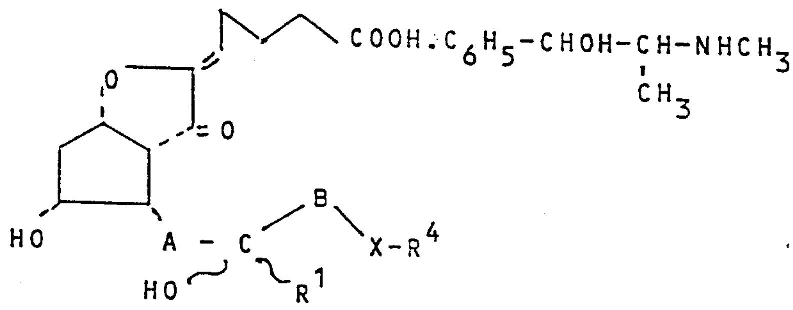

(I) ,

worin
$R^1$, $R^2$, $R^3$, $R^4$, A, B und X die oben angegebene Bedeutung haben,
dadurch gekennzeichnet, daß man

a) aus einer Verbindung der allgemeinen Formel II

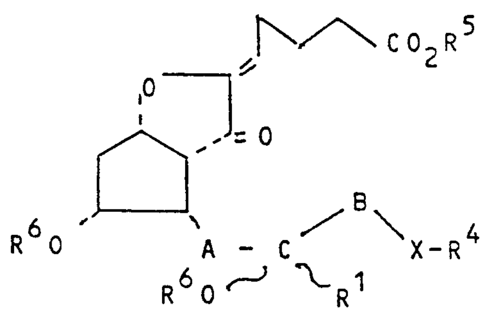

(II) ,

worin
$R^1$, $R^2$, $R^3$, $R^4$, A, B und X die oben angegebene Bedeutung haben,
$R^5$ geradkettiges oder verzweigtes $C_{1-4}$-Alkyl und
$R^6$ geradkettiges oder verzweigtes $C_{1-4}$-Alkanoyl, Benzoyl oder substituiertes Benzoyl bedeuten,
zunächst die Schutzgruppen durch Behandlung mit einer Base und danach die Estergruppe durch Behandlung mit einer Base in Gegenwart von Wasser entfernt, und dann das so erhaltene Reaktionsgemisch in die Salzbildungsreaktion mit optisch aktivem oder racemischem Ephedrin bei -20 bis +50 °C einführt,
oder

b) aus einer Verbindung der allgemeinen Formel II,
worin
$R^1$, $R^2$, $R^3$, $R^4$, $R^5$, A, B und X die oben angegebene Bedeutung haben und
$R^6$ Wasserstoff ist,
zunächst die Estergruppe durch Behandlung mit einer Base in Gegenwart von Wasser entfernt, und danach das so erhaltene Reaktionsgemisch nach Behandlung mit einer Säure und nach Überführung in einen Extrakt mit einem mit Wasser nicht mischbaren organischen Lösungsmittel in die Salzbildungsreaktion mit optisch aktivem oder racemischem Ephedrin bei -20 bis +50 °C einführt,
oder

c) eine mit einem polaren Lösungsmittel gebildete Lösung einer Verbindung der allgemeinen Formel II,
worin
$R^1$, $R^2$, $R^3$, $R^4$, A, B und X die oben angegebene Bedeutung haben und
$R^5$ und $R^6$ Wasserstoff bedeuten,
bei einer Temperatur im Bereich von -20 bis 50 °C mit optisch aktivem oder recemischem Ephedrin behandelt und danach die so erhaltene Verbindung der allgemeinen Formel I zur Kristallisation bringt.

5. Verfahren nach Anspruch 4, dadurch gekennzeichnet, daß man zur Salzbildung das optisch aktive oder racemische Ephedrin in einer Menge von 1,05 bis 5,0 Äquivalenten, vorzugsweise von 1,0 bis 1,1 Äquivalenten verwendet.

6. Verfahren nach Anspruch 5, dadurch gekennzeichnet, daß man als Ausgangsmaterial eine Verbindung der allgemeinen Formel II,
worin
$R^1$, $R^2$, $R^3$, $R^4$, A, B und X die oben angegebene Bedeutung haben,
$R^5$ Wasserstoff oder geradkettiges oder verzweigtes $C_{1-4}$-Alkyl und
$R^6$ Wasserstoff, geradkettiges oder verzweigtes $C_{1-4}$-Alkanoyl, Benzoyl oder substituiertes Benzoyl bedeuten,
verwendet.

7. Pharmazeutisches Präparat, das als Wirkstoff eine oder mehrere Verbindungen der Ansprüche 1 bis 3 enthält.

8. Verfahren zur Herstellung von pharmazeutischen Präparaten gemäß Anspruch 7, dadurch gekennzeichnet, daß man die als Wirkstoff verwendeten Verbindungen der Ansprüche 1 bis 3 mit geeigneten, inerten, festen oder flüssigen pharmazeutischen Trägern vermischt und in die für den therapeutischen Gebrauch geeigneten Präparateformen überführt.

## Claims

1. 7-Oxo-$PGI_2$-ephedrine salt analogs of the general formula I

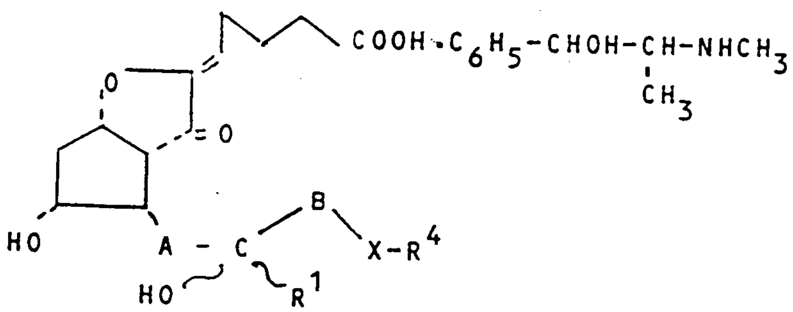

(I) ,

wherein
A denotes $-(CH_2)_2-$, cis- or trans-CH = CH- or -C≡C-,
$R^1$ denotes $C_{1-4}$-alkyl or hydrogen,
B denotes a valency bond, $-CH_2-$ or $-CR^2R^3$,
$R^2$ and $R^3$ denote hydrogen or $C_{1-4}$-alkyl,
X denotes a valency bond, oxygen or $-CH_2-$, and
$R^4$ denotes $C_{1-6}$-alkyl, $C_{4-7}$-cycloalkyl, $C_{1-6}$-ω-fluoroalkyl, $C_{2-6}$-alkenyl, $C_{2-6}$-alkynyl, phenyl or substituted phenyl.

2. 7-Oxo-$PGI_2$-(-)-ephedrine salt, 7-oxo-$PGI_2$-(+)-ephedrine salt or 7-oxo-$PGI_2$-(±)-ephedrine salt.

3. 7-Oxo-16,17,18,19,20-pentanor-15-cyclopentyl-$PGI_2$-(-)-ephedrine salt, 7-oxo-16,17,18,19,20-pentanor-15-cyclopentyl-$PGI_2$-(+)-ephedrine salt or 7-oxo-16,17,18,19,20-pentanor-15-cyclopentyl-$PGI_2$-(±)-ephedrine salt.

4. Process for preparing 7-oxo-$PGI_2$-ephedrine salt analogs of the general formula I

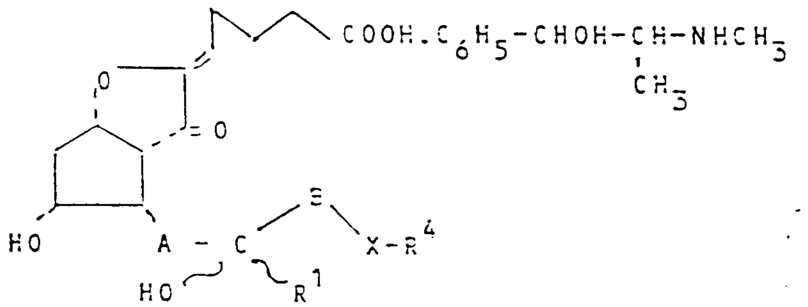

(I) ,

wherein
$R^1$, $R^2$, $R^3$, $R^4$, A, B and X have the meaning given above, characterised in that
a) initially the protective groups are removed from a compound of the general formula II

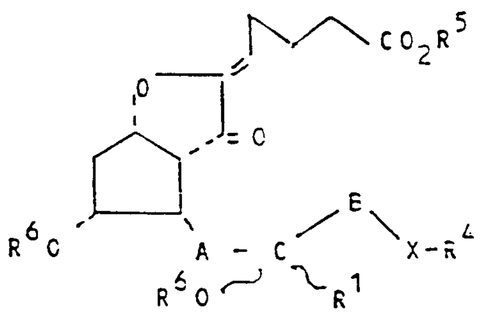

(II) ,

wherein
$R^1$, $R^2$, $R^3$, $R^4$, A, B and X have the meaning given above,
$R^5$ denotes straight-chain or branched $C_{1-4}$-alkyl and
$R^6$ denotes straight-chain or branched $C_{1-4}$-alkanoyl, benzoyl or substituted benzoyl,
by treatment with a base, and then the ester group is removed by treatment with a base in the presence of water, and then the reaction mixture thus obtained is introduced into the salt formation reaction with optically active or racemic ephedrine at -20 to +50°C, or
b) initially the ester group is removed from a compound of the general formula II
wherein
$R^1$, $R^2$, $R^3$, $R^4$, $R^5$, A, B and X have the meaning given above and
$R^6$ is hydrogen,
by treatment with a base in the presence of water, and then the reaction mixture thus obtained is introduced into the salt formation reaction with optically active or racemic ephedrine at -20 to +50°C after treatment with an acid and after conversion to an extract using an organic solvent which cannot be mixed with water, or
c) a solution of a compound of the general formula II formed with a polar solvent,
wherein
$R^1$, $R^2$, $R^3$, $R^4$, A, B and X have the meaning given above and
$R^5$ and $R^6$ denote hydrogen,
is treated with optically active or racemic ephedrine at a temperature in the range from -20 to 50°C, and then the compound of the general formula I thus obtained is crystallised.

**5.** Process according to claim 4, characterised in that the optically active or racemic ephedrine is used in an amount of 1.05 to 5.0 equivalents, preferably 1.0 to 1.1 equivalents, for salt formation.

**6.** Process according to claim 5, characterised in that a compound of the general formula II,
wherein
$R^1$, $R^2$, $R^3$, $R^4$, A, B and X have the meaning given above and
$R^5$ denotes hydrogen or straight-chain or branched $C_{1-4}$-alkyl and
$R^6$ denotes hydrogen, straight-chain or branched $C_{1-4}$-alkanoyl, benzoyl or substituted benzoyl,

is used as starting material.

7. Pharmaceutical preparation which contains one or more compounds of claims 1 to 3 as active ingredient.

8. Process for preparing pharmaceutical preparations according to claim 7, characterised in that the compounds from claims 1 to 3 used as active ingredient are mixed with suitable, inert, solid or liquid pharmaceutical excipients and converted to the preparation forms suitable for therapeutic use.

## Revendications

1. Sels d'éphédrine de la 7-oxo-$PGI_2$ et de composés analogues, répondant à la formule générale I

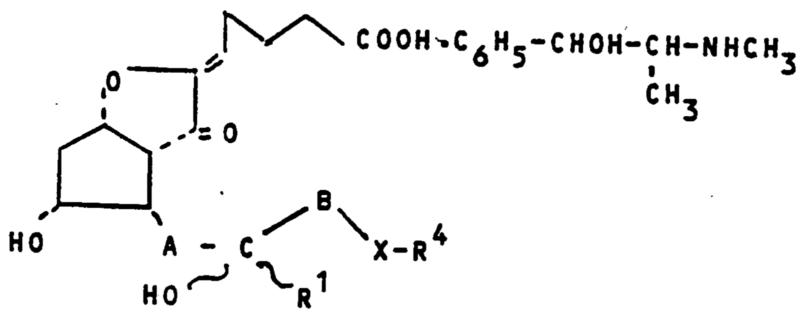

(I) ,

dans laquelle

| | |
|---|---|
| A | représente $-(CH_2)_2-$, cis- ou trans-CH = CH- ou -C≡C-, |
| $R^1$ | représente un groupe alkyle en C 1-C 4 ou l'hydrogène, |
| B | représente une liaison de valence, $-CH_2-$ ou $-CR^2R^3$, |
| $R^2$ et $R^3$ | représentent l'hydrogène ou des groupes alkyle en a 1-C 4, |
| X | représente une liaison de valence, l'oxygène ou $-CH_2-$, et |
| $R^4$ | représente un groupe alkyle en C 1-C 6, cycloalkyle en C 4-C 7, oméga-fluoralkyle en C 1-C 6, alcényle en C 2-C 6, alcynyle en C 2-C 6, phényle ou phényle substitué. |

2. Le sel de (-)-éphédrine de la 7-oxo-$PGI_2$,
le sel de (+)-éphédrine de la 7-oxo-$PGI_2$, ou
le Sel de (±)-éphédrine de la 7-oxo-$PGI_2$.

3. Le sel de (-)-éphédrine de la 7-oxo-16,17, 18,19,20-pentanor-15-cyclopentyl-$PGI_2$,
le sel de (+)-éphédrine de la 7-oxo-16,17,18,19,20-pentanor-15-cyclopentyl-$PGI_2$, ou
le sel de (±)-éphédrine de la 7-oxo-16,17,18,19,20-pentanor-15-cyclopentyl-$PGI_2$.

4. Procédé de préparation de sels d'éphédrine de la 7-oxo-$PGI_2$ et composés analogues, répondant à la formule générale I

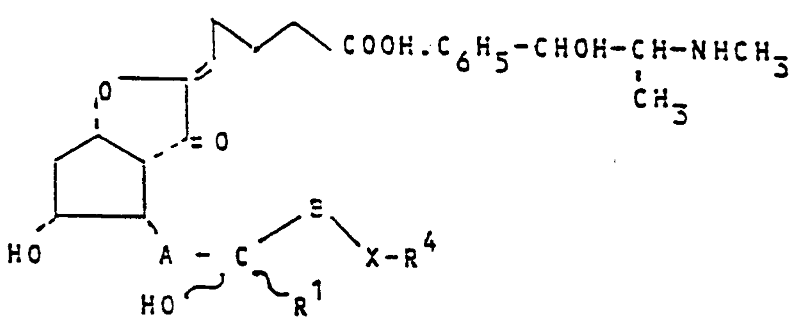

(I) ,

dans laquelle $R^1$, $R^2$, $R^3$, $R^4$, A, B et X ont les significations indiquées ci-dessus, caractérisé en ce que :

a) à partir d'un composé de formule générale II

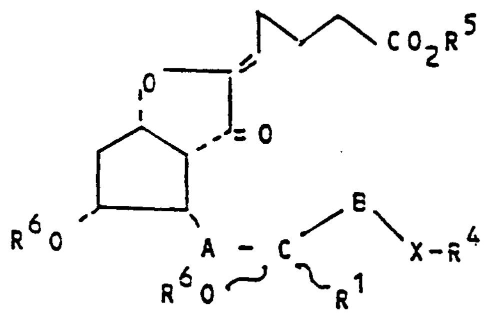

(II) ,

dans laquelle

$R^1$, $R^2$, $R^3$, $R^4$, A, B et X ont les significations indiquées ci-dessus,

$R^5$ représente un groupe alkyle en C 1-C 4 à chaîne droite ou ramifiée, et

$R^6$ représente un groupe alcanoyle en C 1-C 4 à chaîne droite ou ramifiée, benzoyle ou benzoyle substitué,

on élimine d'abord les groupes protecteurs par traitement à l'aide d'une base puis le groupe ester par traitement à l'aide d'une base en présence d'eau, puis on soumet le mélange de réaction à la salification par l'éphédrine énantiomère ou racémique à des températures de -20 à +50 °C, ou bien

b) partant d'un composé de formule générale II dans laquelle

$R^1$, $R^2$, $R^3$, $R^4$, $R^5$, A, B et X ont les significations indiquées ci-dessus, et

$R^6$ représente l'hydrogène,

on élimine d'abord le groupe ester par traitement à l'aide d'une base en présence d'eau, puis le mélange réactionnel ainsi obtenu est introduit dans la réaction de salification, avec de l'éphédrine optiquement active ou racémique à des températures de -20 °C à +50 °C, après traitement avec un acide et après conversion en un extrait à l'aide d'un solvant organique non miscible à l'eau, ou bien

c) on traite une solution, formée avec un solvant polaire, d'un composé de formule générale II dans laquelle

$R^1$, $R^2$, $R^3$, $R^4$, A, B et X ont les significations indiquées ci-dessus, et

$R^5$ et $R^6$ représentent l'hydrogène,

à une température dans l'intervalle de -20 à +50 °C,

par l'éphédrine énantiomère ou racémique puis on fait cristalliser le composé obtenu répondant à la formule générale I.

**5.** Procédé selon la revendication 4, caractérisé en ce que, pour la salification, on utilise l'éphédrine énantiomère ou racémique en quantité de 1,05 à 5,0 équivalents, de préférence de 1,0 à 1,1 équivalent.

**6.** Procédé selon la revendication 5, caractérisé en ce que l'on utilise en tant que produit de départ un composé de formule générale II dans laquelle

$R^1$, $R^2$, $R^3$, $R^4$, A, B et X ont les significations indiquées ci-dessus,

$R^5$ représente l'hydrogène ou un groupe alkyle en C 1-C 4 à chaîne droite ou ramifiée,

$R^6$ représente l'hydrogène, un groupe alcanoyle en C 1-C 4 à chaîne droite ou ramifiée, benzoyle ou benzoyle substitué.

**7.** Composition pharmaceutique contenant un ou plusieurs composés des revendications 1 à 3 en tant que substances actives.

**8.** Procédé de préparation des compositions pharmaceutiques de la revendication 7, caractérisé en ce que l'on mélange les composés des revendications 1 à 3 utilisés en tant que substances actives avec des véhicules inertes, solides ou liquides, appropriés à l'usage pharmaceutique et on met sous la

forme de compositions appropriées à l'usage thérapeutique.